# DEMANDE DE BREVET EUROPEEN

(11) **EP 1 210 913 A1**
(43) Date de publication de la demande: **05.06.2002**
(21) Numéro de dépôt: 02003942.6
(22) Date de dépôt: 24.08.1998
(51) Int. Cl.: A61B 17/32, A61M 5/32

(54) **Dispositif de protection pour un outil perforant**

(30) Priorité: 25.08.1997 FR 9710627
(62) Demande divisionnaire de: 98942771.1
(71) Demandeur: Mosseri, Raphael, 75008 Paris (FR)
(72) Inventeur: Mosseri, Raphael, 75008 Paris (FR)
(74) Mandataire: Abello, Michel

(57) **Abrégé**

Dispositif de protection intégré pour un outil perforant (A), comportant
- un moyen d'isolation (114) pour isoler de l'extérieur une pointe (12) de l'aiguille, ce moyen étant relié à un embout (11) de raccordement à une seringue ;
- un moyen élastique (113) pour rappeler la pointe dans sa position protégée, caractérisé par le fait qu'il comporte un moyen de blocage anti-retour pour bloquer la pointe de l'aiguille dans une position protégée bloquée, ledit moyen de blocage étant maintenu dans sa position inactive par l'aiguille ou le tube contre lequel il prend appui dans la position protégée, ledit moyen de blocage étant apte à elastiquement coopérer avec le tube, à partir de la position protégée, par coulissement du tube par rapport à la pointe de l'aiguille dans un sens opposé au déplacement vers sa position de travail, sous l'action d'une force extérieure jusqu'à la position protégée bloquée, pour interdire le déplacement de la pointe vers sa position de travail.

## Description

La présente invention concerne un dispositif de protection pour un outil perforant, notamment pour un instrument médical tel qu' une aiguille de seringue.

Dans le domaine médical, de nombreux instruments perforants sont utilisés par le personnel soignant, les infirmières, les médecins, les chirurgiens et toute personne environnante, qui peuvent se blesser au cours de leur manipulation.

On peut distinguer d'une part les risques et d'autre part les conséquences.

Les risques actuels encourus lors de l'utilisation d'un instrument perforant, tel qu'une aiguille de seringue, peuvent engendrer une piqûre accidentelle du personnel soignant
- soit par une aiguille encore stérile après ouverture de l'emballage,
- soit par une aiguille ayant déjà été utilisée avec risque d'inoculation d'un produit sanguin (par un instrument soit déjà posé sur la table soit en cours de manipulation).

Les conséquences sont
sur le plan général, pour l'aiguille, un risque de transmission d'un agent infectieux, viral (virus Hépatite, virus HIV,...), bactérien ou parasitaire. Certaines atteintes peuvent être irréversibles.

Tout accident entraîne sa déclaration avec la mise en place de tout un protocole qui peut s'étendre sur plusieurs années et l'indemnisation de cet accident peut grever de façon importante le budget de la Santé Publique.

Le problème lié à 1a protection des personnes utilisant des outils coupants et/ou perforants est particulièrement sensible dans le monde médical car il peut avoir des conséquences dramatiques sur la santé humaine.

Toutefois, malgré la gravité de ce problème et l'existence de maladies très infectieuses depuis de nombreuses années, aucune solution fiable et efficace n'a encore été trouvée pour surmonter ces inconvénients.

On connaît par le brevet US-A-4 850 996 un dispositif conforme au préambule de la revendication 1. Toutefois, ce dispositif ne comporte pas de moyen de blocage anti-retour.

La présente invention a donc pour but d'éliminer les inconvénients précités, de répondre aux exigences de la Santé Publique et de proposer un dispositif de protection générale pour un outil perforant, et plus particulièrement pour un instrument médical.

A cet effet, l'invention a pour objet un dispositif de protection d'un outil perforant, selon la revendication 1. :
L'invention peut comporter les caractéristiques additionnelles suivantes :
   - le moyen de blocage est relié à l'embout de l'aiguille et une portion proximale du tube est apte à franchir ce moyen de blocage lors du coulissement du tube par-rapport à la pointe dans un sens opposé au déplacement vers sa position de travail, pour obtenir un enclenchement élastique du moyen de blocage contre ladite portion proximale du tube ;
   - le moyen élastique est un ressort hélicoïdal monté axialement autour de l'aiguille et reliant l'embout de l'aiguille à une portion proximale du tube et en ce que le ressort est encagé dans une enveloppe de protection supplémentaire fixée à l'embout de l'aiguille et entourant à coulissement le tube précité ;
   - le moyen de blocage est solidaire de l'enveloppe et apte à coopérer avec une collerette proximale du tube ;
   - le moyen élastique est un ressort hélicoïdal qui vient de moulage, à une extrémité, avec le tube précité, et à son autre extrémité opposée, avec un manchon dans lequel vient s'emboîter l'embout de raccordement de l'aiguille ;
   - le moyen de blocage est relié au tube et apte à venir élastiquement s'engager sur l'extrémité distale du tube pour obturer le passage de la pointe vers sa position de travail, lors du coulissement du tube par rapport à la pointe dans un sens opposé au déplacement vers sa position de travail ;
   - ledit tube présentant à son extrémité distale une surface inférieure plane annulaire anti-dérapante, constituée par exemple de rugosités ou de picots et/ou revêtue de matière adhésive, ladite surface annulaire servant aussi pour la visée et le ciblage du vaisseau ou de l'espace corporel par l'aiguille ;
   - le tube présente un marquage longitudinal, par exemple une serrure en saillie ou une lignée colorée, agencé latéralement pour ne pas masquer la visibilité de l'aiguille et la périphérie du vaisseau ou de l'espace corporel à atteindre, afin d'encadrer la direction générale des bordures du vaisseau ou de l'espace corporel.

L'invention sera mieux comprise, et d'autres buts, détails, avantages et caractéristiques de celle-ci apparaîtront plus clairement au cours de la description explicative détaillée qui va suivre de plusieurs modes de réalisation particuliers actuellement préférés de l'invention, donnés uniquement à titre illustratif et non limitatif, en référence aux dessins schématiques annexés, dans lesquels :
- la figure 1 est une vue schématique en coupe axiale d'un dispositif de protection pour une aiguille,
- la figure 2 est une vue analogue à la figure 1, mais représentant l'aiguille dans sa position de travail ;
- la figure 3 est une vue analogue à la figure 1, mais représentant une variante de réalisation d'un dispositif de protection pour une aiguille conformément à l'invention ;
- les figures 4 et 5 sont des vues analogues à la figure 3, mais représentant respectivement l'aiguille dans sa position de travail et dans sa position bloquée en position protégée ;

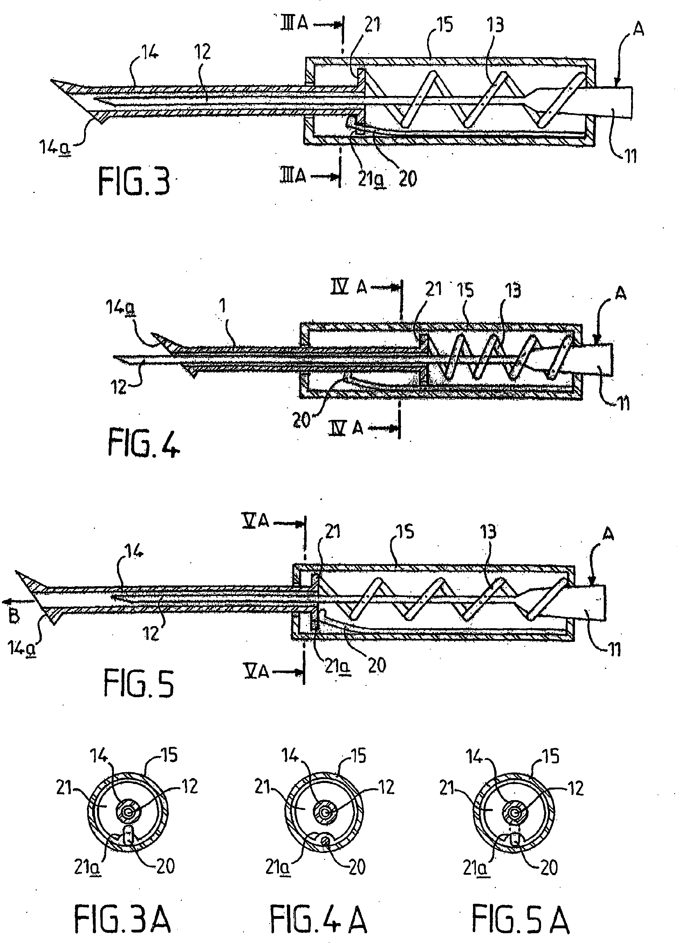

- les figures 3A, 4A et 5A sont des vues en coupe transversale suivant les flèches III-A, IV-A, et V-A des figures 3 à 5 respectivement ;

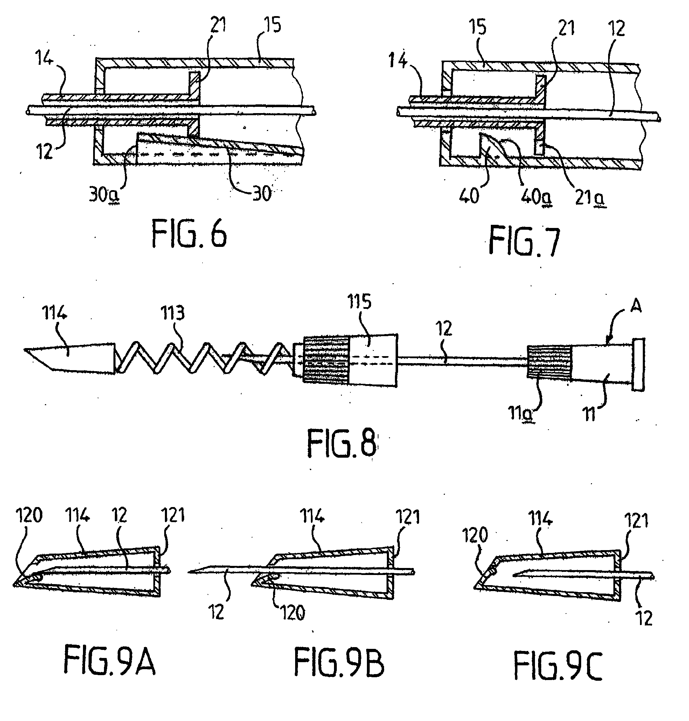

- les figures 6 et 7 sont des vues partielles en coupe axiale de deux autres variantes d'un dispositif de protection pour une aiguille selon l'invention ;
- la figure 8 est une vue éclatée d'un autre mode de réalisation du dispositif de protection pour une aiguille selon l'invention ; et
- les figures 9A à C sont des vues partielles et en coupe axiale du dispositif de la figure 8, respectivement dans une position protégée avant utilisation, dans une position de travail et dans un état bloqué en position protégée .

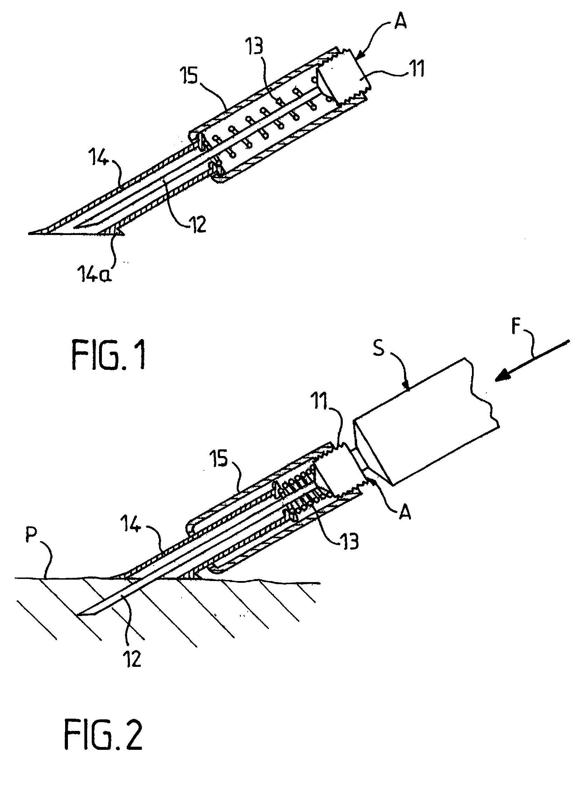

Un dispositif de protection pour une aiguille sera maintenant décrit en référence aux figures 1 et 2, sans moyen de blocage anti-retour.

L'outil perforant est ici une aiguille A pour seringue S, cette aiguille comportant à sa portion proximale un embout tubulaire cylindrique élargi 11 se prolongeant par une portion distale tubulaire effilée de plus petit diamètre 12. Cette portion distale perforante 12 est logée coaxialement coulissante dans un tube transparent de protection 14 qui est relié à son extrémité proximale par un ressort hélicoïdal 13 à l'embout 11 de l'aiguille. De préférence, ce tube 14 est en matière semi-rigide. L'extrémité distale 14a du tube 14 est avantageusement biseautée pour permettre une perforation de la peau P d'un utilisateur dans une direction inclinée comme cela est habituellement le cas. Cette extrémité distale 14a est également renforcée pour résister à la mise en appui du tube 14 contre la peau P lors de la pénétration de la pointe de l'aiguille 12.

Pour éviter tout tâtonnement, il est également important que l'aiguille soit positionnée précisément et correctement lorsque l'on vient piquer la peau pour accéder à un vaisseau ou à un espace corporel.

A cet effet, l'on peut prévoir à l'extrémité distale 14a du tube 14, une surface inférieure plane annulaire anti-dérapante, constituée par exemple de rugosités ou de picots et/ou revêtue de matière adhésive. Ainsi, lors de la mise en appui du tube contre la peau, le tube ne risquera pas de glisser et d'entraver la précision de la piqûre.

En outre, cette surface annulaire distale facilitera la visée et le ciblage du vaisseau ou de l'espace corporel par l'aiguille. En effet, avant même de piquer la peau, l'opérateur pourra réajuster sa visée par manipulations successives du dispositif.

On peut aussi en variante prévoir un marquage le long du tube, par exemple une nervure en saillie ou une ligne colorée, agencé latéralement pour ne pas masquer la visibilité de l'aiguille et de la périphérie du vaisseau ou de l'espace corporel à atteindre, afin d'encadrer la direction générale des bordures du vaisseau ou de l'espace corporel.

L'inclinaison de la surface est avantageusement de 45° par rapport à l'axe de l'aiguille pour les prélèvements vasculaires ou perpendiculaire à celle-ci pour accéder dans un espace rachidien par exemple.

On notera enfin que ce dispositif est un dispositif de sécurité intégré automatique et permanent, à savoir dès la sortie de l'aiguille de son emballage, avant, pendant et après la manipulation. Ce dispositif de sécurité n'a pas à être activé par l'opérateur.

On peut également prévoir une enveloppe externe de protection 15 reliée à son extrémité proximale à l'embout 11 de l'aiguille A et montée coulissante autour du tube 14 à son extrémité distale. Cette enveloppe 15 permet d'isoler de l'extérieur le ressort 13 et peut également servir au guidage du tube 14 au cours de son déplacement.

Le fonctionnement du dispositif illustré sur les figures 1 et 2 sera maintenant brièvement expliqué.

Dans la position de repos illustrée sur la figure 1, la portion perforante ou pointe 12 de l'aiguille A est complètement escamotée à l'intérieur du tube 14, lequel tube 14 présente ne longueur inférieure à celle de la portion distale 12.

Lorsqu'un utilisateur vient appliquer l'extrémité distale 14a du tube 14 contre la peau P d'un patient, comme illustré sur la figure 2, il exerce une pression dans la direction axiale F du dispositif au niveau de l'embout 11 de l'aiguille A, par exemple par l'intermédiaire d'une seringue S, ce qui a pour effet de comprimer le ressort 13 et de faire coulisser la portion perforante 12 de l'aiguille dans le tube 14 jusqu'à ce que ladite portion perforante émerge du tube et pénètre dans la peau P. On comprendra que dès que la pression est relâchée au niveau de la seringue, l'aiguille 12 sera rappelée dans sa position protégée par le ressort de rappel 13.

Sur les figures 3 à 5, on a représenté une variante de réalisation selon l'invention dans laquelle l'enveloppe 15 est munie sur sa paroi latérale interne d'une patte de blocage 20 élastiquement déformable. Cette patte de blocage 20 est fixée à une extrémité à la paroi interne de l'enveloppe 15 et présente à son extrémité libre opposée une partie recourbée apte à coopérer avec une collerette 21 formée à l'extrémité proximale du tube 14. Cette collerette 21 qui s'étend radialement vers l'extérieur coulisse à l'intérieur de l'enveloppe 15 et comporte à sa périphérie un renfoncement 21a pour le passage de l'extrémité libre de la patte de blocage 20.

Le fonctionnement du dispositif illustré sur les figures 3 à 5 va maintenant être décrit. Dans la position illustrée sur les figures 3 et 3A, le tube 14 est sollicité vers sa position de protection de la pointe 12 de l'aiguille, par le ressort 13, la collerette 21 du tube 14 venant buter contre l'extrémité recourbée de la patte de blocage 20 qui traverse le renfoncement 21a de la collerette 21.

Le tube 14 est amené dans sa position de travail illustrée sur les figures 4 et 4A, en venant appuyer sa portion distale élargie 14a contre la peau de l'utilisateur, pour découvrir la pointe 12 de l'aiguille. La collerette 21 du tube 14 contre laquelle prend appui le ressort 13 vient comprimer ce dernier et coulisse à l'intérieur de l'enveloppe 15. La collerette 21 coulisse sur la patte de blocage 20 en direction de son extrémité fixe.

Lorsque le travail est terminé, le tube 14 revient dans sa position illustrée sous la figure 3, sous la force de rappel du ressort 13.

Puis, pour rendre le dispositif non réutilisable, l'opérateur exerce une force de traction sur le tube 14, dans la direction de la flèche B sur la figure 5, jusqu'à ce que la collerette 21 franchisse l'extrémité libre recourbée de la patte de blocage 20, laquelle patte 20 fléchit légèrement vers l'extérieur, puis revient élastiquement vers l'intérieur pour venir buter contre la collerette 21, au-dessus du renfoncement 21a, empêchant ainsi tout mouvement de retour du tube 14 pour découvrir la pointe 12, comme visible sur la figure 5A.

Bien que cela ne soit pas illustré, on pourrait remplacer la patte de blocage 20, par un moyen de blocage anti-retour destiné à coopérer avec la collerette 21, par une rotation relative du tube 14, par rapport à l'enveloppe 15, un moyen d'encliquetage étant prévu entre la périphérie de la collerette 21 et la paroi intérieure de l'enveloppe 15.

Dans la variante de la figure 6, la patte de blocage 20 est remplacée par une rampe inclinée 30 qui vient de moulage avec l'enveloppe 15, la rampe 30 s'étendant vers l'intérieur de l'enveloppe 15 à partir de sa portion proximale vers sa portion distale. La collerette 21 est apte à coulisser par son renfoncement 21a sur la rampe 30 qui présente un profil complémentaire. La rampe 30 est ouverte à sa portion distale, pour des contraintes de démoulage. Cette rampe 30 est élastiquement déformable, lors de son franchissement par la collerette 20, pour amener le tube 14 dans sa position protégée bloquée.

Dans une autre variante illustrée sur la figure 7, la rampe 30 est remplacée par une dent 40 qui présente un profil incurvé 40a tourné vers le renfoncement 21a de la collerette 20, pour permettre son franchissement, et une face opposée parallèle au plan de la collerette 21 pour servir de surface d'appui anti-retour, dans la position protégée de blocage du tube 14. Cette dent 40 est également élastiquement déformable lors de son franchissement par la collerette 21.

On pourrait également, sans sortir du cadre de l'invention, prévoir la collerette 21 élastiquement déformable, au lieu de la rampe 30 ou de la dent 40.

Dans un autre mode de réalisation illustré sur la figure 8, le dispositif de l'invention comprend un tube 114, dont la portion proximale est liée à un ressort 113 qui est à son tour relié par sa spire d'extrémité proximale à un manchon 115. Les éléments 113 à 115 sont monoblocs et viennent de moulage. L'embout 11 de l'aiguille A est destiné à s'emboîter dans le manchon 115, l'embout 11 comportant sur sa paroi périphérique des cannelures 11a qui sont destinées à s'engager dans des cannelures correspondantes formées sur la paroi interne du manchon 115, avec un ajustement serré pour leur raccordement.

Comme visible sur les figures 9A à 9C, le tube 114 comporte au niveau de sa portion distale une patte 120 qui est élastiquement repliée vers l'intérieur du tube 114 et maintenue dans cette position par la pointe 12 de l'aiguille. A sa portion proximale, le tube 114 comporte une collerette radialement saillante vers l'intérieur 121, à travers laquelle passe la pointe 12 de l'aiguille A pour son guidage.

Dans la position illustrée sur la figure 9A, la pointe 12 de l'aiguille A est protégée par le tube 114 et retient la patte de blocage 120 dans une position inactive. Lorsque la pointe 12 de l'aiguille est amenée dans sa position de travail, la pointe 12 glisse sur la patte de blocage 120 qui est toujours maintenue dans une position inactive.

Après utilisation, pour rendre le dispositif non réutilisable, l'utilisateur exerce une traction sur le tube 114, pour éloigner la pointe 12 de l'aiguille de la patte 120 qui est élastiquement rappelée dans une position où elle obture le passage du tube 114, à son extrémité distale, interdisant ainsi la sortie de la pointe 12 hors du tube 114.

Il est clair pour l'homme du métier que l'invention n'est pas limitée à une aiguille, mais peut s'appliquer à tout autre élément perforant, dans des domaines autres que la médecine. Il est également clair que l'invention n'est limitée ni à la forme ni à la structure des dispositifs illustrés, toute autre forme ou structure permettant d'atteindre les mêmes fonctions faisant partie du cadre de la présente invention.

## Revendications

1. Dispositif de protection intégré pour un outil perforant (A), comportant
- une aiguille (A) constituant ledit outil perforant,
- un moyen d'isolation (14, 114) pour isoler de l'extérieur une pointe (12) formant la partie distale perforante de l'aiguille, ce moyen étant relié à un embout (11) de raccordement à une seringue (S) formant la partie proximale de l'aiguille avec une mobilité relative limitée et présentant une ouverture pour le passage de ladite pointe entre une position protégée, dans laquelle ledit moyen entoure au moins partiellement ladite pointe, et une position de travail, dans laquelle ladite pointe est dégagée dudit moyen,
- un moyen élastique (13, 113) pour rappeler la pointe dans sa position protégée, ladite pointe étant apte à se déplacer vers sa position de travail sous l'effet d'une pression externe (F) s'opposant à la force de rappel élastique, ledit moyen d'isolation étant constitué d'un tube transparent (14, 114) ouvert à ses deux extrémités, à travers lequel est montée coulissante la partie distale (12) de l'aiguille, **caractérisé par le fait qu'**il comporte un moyen de blocage anti-retour (20, 30, 40, 120) pour bloquer la pointe de l'aiguille dans une position protégée bloquée, ledit moyen de blocage étant maintenu dans sa position inactive par l'aiguille ou le tube contre lequel il prend appui dans la position protégée, ledit moyen de blocage étant apte à elastiquement coopérer avec le tube, à partir de la position protégée, par coulissement du tube par rapport à la pointe (12) de l'aiguille dans un sens opposé au déplacement vers sa position de travail, sous l'action d'une force extérieure jusqu'à la position protégée bloquée, pour interdire le déplacement de la pointe vers sa position de travail.

2. Dispositif selon la revendication 1, **caractérisé en ce que** le moyen de blocage (20, 30, 40) est relié à l'embout (11) de l'aiguille et une portion proximale (21) du tube (14) est apte à franchir ce moyen de blocage lors du coulissement du tube vers sa position protégée bloquée, pour obtenir un enclenchement élastique du moyen de blocage contre ladite portion proximale du tube.

3. Dispositif selon la revendication 1 ou 2, **caractérisé en ce que** le moyen élastique est un ressort hélicoïdal (13) monté axialement autour de l'aiguille et reliant l'embout (11) de l'aiguille à une portion proximale (21), du tube (14) et **en ce que** le ressort est encagé dans une enveloppe de protection supplémentaire (15) fixée à l'embout (11) de l'aiguille et entourant à coulissement le tube précité (14).

4. Dispositif selon la revendication3, **caractérisé en ce que** le moyen de blocage (20, 30, 40) est solidaire de l'enveloppe (15) et apte à coopérer avec une collerette proximale (21) du tube (14).

5. Dispositif selon la revendication 1, **caractérisé en ce que** le moyen élastique est un ressort hélicoïdal (113) qui est monobloc, à une extrémité, avec le tube précité (114), et à son autre extrémité opposée, avec un manchon (115) dans lequel vient s'emboîter l'embout de raccordement (11) de l'aiguille.

6. Dispositif selon la revendication 5, **caractérisé en ce que** le moyen de blocage (120) est relié au tube (114) et apte à venir élastiquement s'engager sur l'extrémité distale du tube pour obturer le passage de la pointe vers sa position de travail, lors du coulissement du tube vers sa position protégée bloquée.

7. Dispositif selon l'une des revendications 1 à 6, **caractérisé en ce que** ledit tube (14, 114) présentant à son extrémité distale (14a) une surface inférieure plane annulaire anti-dérapante, constituée par exemple de rugosités ou de picots et/ou revêtue de matière adhésive, ladite surface annulaire (14a) servant aussi pour la visée et le ciblage du vaisseau ou de l'espace corporel par l'aiguille.

8. Dispositif selon l'une des revendications de 1 à 7, **caractérisé en ce que** le tube (14, 114) présente un marquage longitudinal, par exemple une serrure en saillie ou une lignée colorée, agencé latéralement pour ne pas masquer la visibilité de l'aiguille et la périphérie du vaisseau ou de l'espace corporel à atteindre, afin d'encadrer la direction générale des bordures du vaisseau ou de l'espace corporel.
